# EUROPÄISCHE PATENTSCHRIFT

(11) **EP 0 378 046 B1**
(45) Veröffentlichungstag und Bekanntmachung des Hinweises auf die Patenterteilung: **18.01.1995**
(21) Anmeldenummer: 89810918.6
(22) Anmeldetag: 05.12.1989
(51) Int. Cl.: C07D 207/34, C07D 405/04, A01N 43/36

(54) **Verfahren zur Herstellung von 3-Phenylpyrrolderivaten**
Process for the preparation of 3-phenyl-pyrrole derivatives
Procédé de préparation de dérivés de 3-phényl-pyrrole

(30) Priorität: 12.12.1988 US 283341; 11.10.1989 US 419793
(43) Veröffentlichungstag der Anmeldung: 18.07.1990
(73) Patentinhaber: CIBA-GEIGY AG, 4002 Basel (CH)
(72) Erfinder: Pfluger, Rudolf Walter, Dr., CH-4314 Zeiningen (CH); Indermühle, Jean, Dr., CH-4058 Basel (CH); Felix, Franz, Dr., CH-4142 Münchenstein (CH)

(56) Entgegenhaltungen:
- EP-A- 0 111 452
- EP-A- 0 133 247
- EP-A- 0 206 999
- JP-A- 6 130 571
- TETRAHEDRON LETTERS, Nr. 52, Dezember 1972, Seiten 5337-5340, Pergamon Press,Oxford, GB; A.M. VAN LEUSEN et al.: "A new and simple synthesis of the pyrrolering system from Michael acceptors and tosylmethylisocyanides"
- ORGANIKUM, Organisch-Chemisches Grundpraktikum, 9., überarbeitete Auflage, 1970, Seiten 598,603, Veb Deutscher Verlag der Wissenschaften, Berlin, DE
- SYNTHESIS-JOURNAL OF SYNTHETIC ORGANIC CHEMISTRY, Nr. 4, April 1985, Seiten 400-402, Georg Thieme Verlag, Stuttgart, DE; R. OBRECHT et al.: "Isocyanide synthesis with phosphoryl chloride and diisopropylamine"
- ORGANIC SYNTHESES, Band 57, 1978, Seiten 102-106, John Wiley and Sons, NewYork, US; B.E. HOOGENBOOM et al.: "p-Tolylsulfonylmethyl isocyanide [Benzene,1-((isocyanomethyl)sulfonyl)-methyl-]"
- JOURNAL OF ORGANIC CHEMISTRY, Band 42, Nr. 7, 1. April 1977, Seiten 1153-1159; A.M. VAN LEUSEN et al.: "Base-induced cycloaddition of sulfonylmethylisocyanides to C,N double bonds. Synthesis of 1,5-disubstituted and 1,4,5-trisubstituted imidazoles from aldimines and imidoyl chlorides"

## Beschreibung

Die vorliegende Erfindung betrifft ein neues Verfahren zur Herstellung von 3-Phenylpyrrolderivaten der Formel I 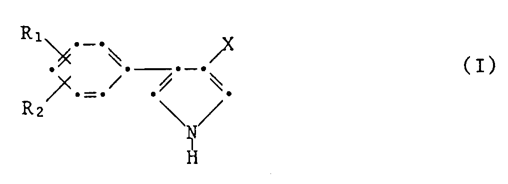
worin X für Cyano, -CO-R₃, -CO-OR₃ oder 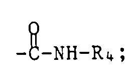
R₁ und R₂ unabhängig voneinander für Wasserstoff, C₁-C₆-Alkyl, C₁-C₆-Alkoxy, C₁-C₆-Alkylthio, Nitro, Cyano, Halogen oder C₁-C₆-Halogenalkyl, oder
R₁ und R₂ zusammen für Methylendioxy oder Difluormethylendioxy stehen;
R₃ C₁-C₆-Alkyl, C₁-C₆-Halogenalkyl, Phenyl oder Benzyl oder durch Halogen, Methyl, Methoxy oder Methylthio substituiertes Phenyl oder Benzyl bedeutet;
R₄ für Wasserstoff, C₁-C₆-Alkyl, C₁-C₆-Halogenalkyl, Phenyl oder Benzyl oder durch Halogen, Methyl, Methoxy oder Methylthio substituiertes Phenyl oder Benzyl steht.

Unter Alkyl ist geradkettiges oder verzweigtes Alkyl zu verstehen. Geeignete C₁-C₆-Alkylreste sind beispielsweise: Methyl, Aethyl, n-Propyl, i-Propyl, n-Butyl, i-Butyl, sek.-Butyl, tert.-Butyl, n-Pentyl oder die isomeren Pentyl, Hexyl oder die isomeren Hexyl. Bevorzugt sind Alkylreste mit höchstens 4 Kohlenstoffatomen.

Unter Halogen selbst sowie als Teil eines Substituenten wie in Halogenbenzyl, Halogenphenyl oder Halogenalkyl sind Fluor, Chlor oder Brom, vorzugsweise jedoch Fluor oder Chlor zu verstehen. Halogenalkyl steht in der Regel für Chlormethyl, Fluormethyl, Difluormethyl, Trifluormethyl, 2-Chloräthyl, 2,2,2-Trifluoräthyl, 1,1,2,2-Tetrafluoräthyl, Pentafluoräthyl, 1,1,2-Trifluor-2-chloräthyl, 2,2,2-Trifluor-1,1-dichloräthyl, Pentachloräthyl, 3,3,3-Trifluorpropyl, 2,3-Dichlorpropyl, 1,1,2,3,3,3-Hexafluorpropyl, 1-Chlorpentyl, 1-Chlorhexyl, insbesondere aber für Fluormethyl, Chlormethyl, Difluormethyl oder Trifluormethyl.

Substituierte Benzyl- und Phenylgruppen sind beispielsweise: 2-Chlorphenyl, 3-Chlorphenyl, 4-Chlorphenyl, 2,4-Dichlorphenyl, 3,4-Dichlorphenyl, 2,3-Dichlorphenyl, 2,4,6-Trichlorphenyl, 2,5-Dichlorbenzyl, 4-Fluorphenyl, 2-Fluorbenzyl, 4-Methylphenyl, 2,4-Dimethylphenyl, 4-Trifluormethylphenyl, 4-Brombenzyl, 4-Methoxyphenyl, 2,4-Dimethoxyphenyl und 4-Methylthiophenyl.

Unter Alkoxy ist zu verstehen: Methoxy, Aethoxy, n-Propyloxy, i-Propyloxy oder die vier isomeren Butyloxy, n-Pentyloxy oder die isomeren Pentyloxy, n-Hexyloxy oder die isomeren Hexyloxy, insbesondere aber Methoxy, Aethoxy oder i-Propyloxy.

Beispiele für Alkylthio sind Methylthio, Aethylthio, n-Propylthio, i-Propylthio oder die vier isomeren Butylthio, n-Pentylthio oder die isomeren Pentylthio, n-Hexylthio oder die isomeren Hexylthio, insbesondere aber Methylthio.

Ein 3-Phenylpyrrolderivat der Formel I, worin R₁ und R₂ zusammen für Difluormethylendioxy stehen und X Cyano bedeutet, ist aus der Europäischen Patentanmeldung EP-A 206999 als mikrobizides Mittel bekannt.

3-Phenylpyrrolderivate der Formel I, worin R₁ und R₂ unabhängig voneinander für Wasserstoff, Halogen, Methoxy oder Methylthio stehen und X Cyano bedeutet, sind aus der europäischen Patentanmeldung EP-A-133 247 als Zwischenprodukte zur Herstellung mikrobizider Mittel bekannt.

Aus der europäischen Patentanmeldung EP-A-236 272 ist bekannt, dass die Verbindung der Formel I, worin R₁ für 2-Chlor, R₂ für 3-Chlor und X für Cyano steht, eine hohe fungizide Aktivität entfaltet.

Nach einem in Tetrahedron Letters (1972) 52, 5337-5340 beschriebenen Verfahren wird Zimtsäurenitril (Formel III) 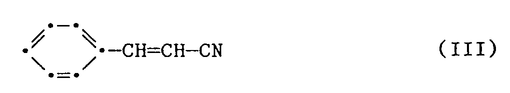
mit (p-Toluolsulfonyl)-methylisocyanid (TOSMIC, Formel IV) 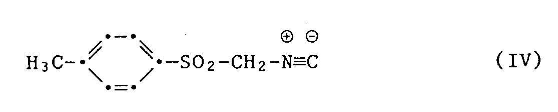
in Gegenwart einer starken Base, wie z.B. Natriumhydrid, zu einem 4-Cyano-3-phenylpyrrol der Formel (V) 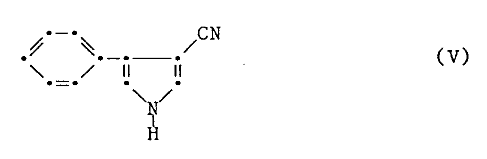
zyklisiert. Neben der für Synthesen im technischen Massstab sehr geringen Ausbeute von nur 35 % besteht ein wesentlicher Nachteil dieses Verfahrens darin, dass es nur mit isoliertem, rekristallisiertem (p-Toluolsulfonyl)-methylisocyanid durchgeführt werden kann. Die Handhabung von isoliertem, rekristallisiertem (p-Toluolsulfonyl)-methylisocyanid ist jedoch mit einem sehr grossen Sicherheitsrisiko verbunden. So neigt die Verbindung bei erhöhten Temperaturen zu explosionsartigem Zerfall.

Ferner macht die erforderliche völlig wasserfreie Reaktionsführung das beschriebene Verfahren zusätzlich kostenintensiv und aufwendig. Durch die genannten Nachteile erweist sich dieses Verfahren für die industrielle Produktion von 3-Phenylpyrrolderivaten der Formel I als nachteilig.

In "Organic Syntheses", Vol. 57, pp. 102-106 [1978] wird eine ausführliche Beschreibung der Gewinnung von p-Toluolsulfonylmethylisocyanid gegeben. Als bevorzugte Base wird Triethylämin angegeben.

In "Synthesis" No. 4, pp. 400-402 [1985] wird beschrieben, dass das Verfahren zur Herstellung von Isocyaniden durch Wasserabspaltung aus entsprechenden Formamiden in Gegenwart von Phosphorylchlorid (Phosphoroxychlorid) besonders vorteilhaft mit Diisopropylamin erfolgt. Das gewonnene Isocyanid kann mit wässrigen Laugen gewaschen werden. Seine Reindarstellung erfolgt aus wasserfreiem Medium.

In der EP-A-111 452 werden sulfenylierte Pyrrolderivate als Biocide für den Materialschutz vorgeschlagen. Der Pyrrol-Ringschluss solcher Produkte wird durch cyclisierende Michael-Addition mit Tosmic durchgeführt. Es wird erwähnt, dass in Gegenwart wässriger Basen gearbeitet werden kann. Beispiele werden für diese Variante nicht gegeben.

Die offengelegte japanische Patentanmeldung Sho 61-30571 beschreibt ein Verfahren zur Herstellung von 4-Cyano-3-phenylpyrrolen der Formel VI 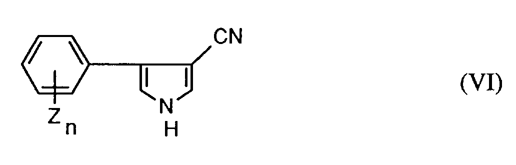
worin Z für Halogen, Alkyl, Halogenalkyl, Alkylamino, Alkoxy, Nitro, Cyano oder Methylendioxy steht und n 0, 1 oder 2 bedeutet, durch Umsetzung eines Cyanozimtsäureesters der Formel VII 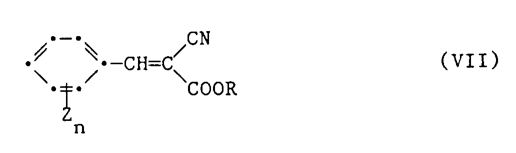
worin Z und n die oben genannte Bedeutung haben und R für Wasserstoff oder Alkyl steht, mit (p-Toluolsulfonyl)-methylisocyanid in Gegenwart einer Base.

Dieses Verfahren weist wie das zuvor beschriebene den schwerwiegenden Nachteil auf, dass zur Synthese der Verbindung der Formel VI in befriedigenden Ausbeuten nur isoliertes, gereinigtes, rekristallisiertes (p-Toluolsulfonyl)-methylisocyanid eingesetzt werden kann. Wie bereits erwähnt, ist kristallines (p-Toluolsulfonyl)-methylisocyanid thermolabil und deflagrationsfähig und stellt wegen akuter Explosionsgefahr bei Synthesen in technischem Massstab eine erhebliche Gefahrenquelle dar.

Es wurde nun ein neues Verfahren gefunden, welches eine gefahrlose und ökonomisch vorteilhafte Herstellung von Verbindungen der Formel I aus (p-Toluolsulfonyl)-methylisocyanid in technischem Massstab in guten Ausbeuten ermöglicht.

Das erfindungsgemässe neue Verfahren zur Herstellung von 3-Phenylpyrrolderivaten der Formel I besteht darin, dass man
a) N-(p-Toluolsulfonyl)-methylformamid in einem inerten Lösungsmittel in Gegenwart einer organischen Base mit Phosphoroxychlorid umsetzt, die Reaktionslösung mit Wasser vermischt, die wässrige Phase des erhaltenen zweiphasigen Gemisches abtrennt, und
b) die (p-Toluolsulfonyl)-methylisocyanid enthaltende organische Phase unmittelbar mit einer Verbindung der Formel II 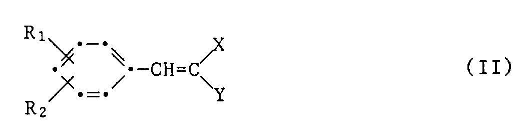 worin X, R₁ und R₂ die unter Formel I angegebene Bedeutung haben;
   Y für -CO-NHR₄, -CO-R₅ oder -S-R₅ steht;
   R₅ für C₁-C₆-Alkyl, C₁-C₆-Halogenalkyl, Phenyl oder Benzyl oder durch Halogen, Methyl, Methoxy oder Methylthio substituiertes Phenyl oder Benzyl steht; und
   R₄ die unter Formel I angegebenen Bedeutung hat, in Gegenwart einer Base umsetzt.

Geeignete inerte Lösungsmittel sind beispielsweise Verbindungen oder Gemische von Verbindungen aus der Gruppe der offenkettigen oder zyklischen Aether wie z.B. Dioxan, Tetrahydrofuran, Diäthyläther, Diisopropyläther, Dimethoxymethan, 1,2-Dimethoxyäthan oder aus der Gruppe der Chloralkane wie z.B. Methylenchlorid, Chloroform oder Tetrachlorkohlenstoff, oder Verbindungen aus der Gruppe der Ketone wie beispielsweise Aceton, Methyläthylketon, Diäthylketon, Cyclohexanon, Methylisopropylketon oder Methylisobutylketon oder aus der Gruppe der Alkylester niederer Carbonsäuren wie Essigsäuremethylester, Essigsäureäthylester oder Propionsäureäthylester. Bevorzugte Lösungsmittel sind Tetrahydrofuran, Dioxan, Dimethoxymethan, Essigsäureäthylester und 1,2-Dimethoxyäthan, besonders bevorzugt sind 1,2-Dimethoxyäthan und Essigsäureäthylester.

Zur Herstellung von (p-Toluolsulfonyl)-methylisocyanid aus N-(p-Toluolsulfonyl)-methylformamid geeignete organische Basen sind beispielsweise Chinolin, Pyridin oder Diisopropylamin, besonders tertiäre Amine wie z.B. Chinuclidin, N,N-Dimethylanilin, Dimethylaminopyridin, N-Methylpyrrolidin oder N,N,N',N'-Tetramethyläthylendiamin, insbesondere aber Trialkylamine wie Triäthylamin oder Tri-n-propylamin; besonders bevorzugt ist Triäthylamin.

Beispiele für geeignete Basen zur Umsetzung von (p-Toluolsulfonyl)-methylisocyanid mit der Verbindung der Formel II sind Oxide, Hydride, Hydroxide, Carbonate, Carbonsäuresalze oder Alkoholate eines Alkali- oder Erdalkalimetalls, Trialkylamine oder Pyridinbasen. Als besonders gut geeignet haben sich Natriummethylat, Natriumäthylat, wässriges oder methanolisches Natriumhydroxid, wässriges oder methanolisches Kaliumhydroxid, Natriumcarbonat oder Kaliumcarbonat erwiesen. Besonders bevorzugt ist wässriges Natriumhydroxid, methanolisches Natriumhydroxid, wässriges Kaliumhydroxid, methanolisches Kaliumhydroxid und Natriummethylat.

In einer bevorzugten Ausführungsform des erfindungsgemässen Verfahrens wird die Verbindung der Formel II gemeinsam mit der (p-Toluolsulfonyl)-methylisocyanidlösung vorgelegt und die Base anschliessend zudosiert.

Ueblicherweise wird die Base bei der Umsetzung der Verbindung der Formel II mit (p-Toluolsulfonyl)-methylisocyanid in einer Menge von 2 bis 3 Mol pro Mol Verbindung der Formel II eingesetzt, bevorzugt ist eine Menge von 2 bis 2,4 Mol Base pro Mol Verbindung der Formel II. Die Umsetzung der Verbindung der Formel II mit (p-Toluolsulfonyl)-methylisocyanid findet in der Regel bei Temperaturen von -25° bis +25°C statt, wobei der Temperaturbereich von -10°C bis +10°C bevorzugt wird.

Die Verbindung der Formel II und das (p-Toluolsulfonyl)-methylisocyanid werden vorteilhaft in äquimolaren Mengen oder mit einem Ueberschuss von 10 bis 20 Mol% an Verbindung der Formel II eingesetzt, besonders bevorzugt sind äquimolare Mengen.

Werden im Verfahrensschritt a) als inerte Lösungsmittel Ketone oder Alkylester niederer Carbonsäuren verwendet, so hat es sich zur Erzielung höheren Ausbeuten als vorteilhaft erwiesen, vor der Durchführung des Verfahrensschritts b) die organische Phase mit einer wässrigen Lösung einer Base zu versetzen und die wässrige Phase anschliessend abzutrennen. Geeignete Basen für diese Verfahrensvariante sind beispielsweise Natriumhydroxid, Kaliumhydroxid, Natriumcarbonat, Natriumhydrogencarbonat und Kaliumcarbonat, insbesondere aber Natriumhydroxid und Natriumcarbonat. Als inertes Lösungsmittel hat sich bei dieser Verfahrensvariante Essigsäureäthylester als besonders gut geeignet erwiesen.

In einer besonders bevorzugten Ausführungsform des erfindungsgemässen Verfahrens wird N-(p-Toluolsulfonyl)-methylformamid in 1,2-Dimethoxyäthan in Gegenwart von Triäthylamin mit Phosphoroxychlorid umgesetzt, die erhaltene Reaktionsmischung mit dem 0,5- bis 0,7-fachen Volumen an Wasser vermischt, die wässrige Phase aus dem so erhaltenen zweiphasigen Reaktionsgemisch abgetrennt und die (p-Toluolsulfonyl)-methylisocyanid enthaltende organische Phase unmittelbar mit einer Verbindung der Formel II in Gegenwart einer Base umgesetzt.

Eine weitere Ausführungsform ist dadurch gekennzeichnet, dass man
a) die Umsetzung von N-(p-Toluolsulfonyl)-methylformamid mit Phosphoroxychlorid in 1,2-Dimethoxyäthan in Gegenwart von Triäthylamin durchführt, die Reaktionsmischung mit dem 0,55- bis 0,6-fachen Volumen an Wasser vermischt, die wässrige Phase aus dem so erhaltenen zweiphasigen Reaktionsgemisch abtrennt, und
b) die (p-Toluolsulfonyl)-methylisocyanid enthaltende organische Phase unmittelbar mit einer äquimolaren Menge am Verbindung der Formel II in bezug auf die Menge an (p-Toluolsulfonyl)-methylisocyanid in Gegenwart von 2 bis 2,4 Mol wässrigem Natriumhydroxid in bezug auf die Verbindung der Formel II bei einer Temperatur von -10°C bis +10°C umsetzt.

In einer weiteren bevorzugten Ausführungsform des erfindungsgemässen Verfahrens wird als inertes Lösungsmittel Dioxan verwendet und die nach Umsetzung von N-(p-Toluolsulfonyl)-methylformamid mit Phosphoroxychlorid erhaltene Reaktionsmischung mit dem 0,2- bis 0,35-fachen Volumen an Wasser vermischt.

In einer anderen Variante des erfindungsgemässen Verfahrens wird als inertes Lösungsmittel Tetrahydrofuran verwendet und die nach Umsetzung von N-(p-Toluolsulfonyl)-methylformamid mit Phosphoroxychlorid erhaltene Reaktionsmischung dem 0,3- bis 0,8-fachen Volumen an Wasser vermischt.

In einer weiteren Variante des erfindungsgemässen Verfahrens wird als inertes Lösungsmittel Dimethoxymethan verwendet und die mach Umsetzung von N-(p-Toluolsulfonyl)-methylformamid mit Phosphoroxychlorid erhaltene Reaktionsmischung mit dem 0,3- bis 1,2-fachen Volumen an Wasser vermischt.

Bevorzugt werden Verbindungen der Formel I eingesetzt, worin R₁ und R₂ unabhängig voneinander für Wasserstoff. Halogen, Methoxy oder Methylthio oder zusammen für Methylendioxy oder Difluormethylendioxy stehen; R₃ und R₅ unabhängig voneinander C₁-C₄-Alkyl oder C₁-C₄-Halogenalkyl bedeuten; R₄ für Wasserstoff, C₁-C₄-Alkyl oder C₁-C₄-Halogenalkyl steht und Y -CO-NHR₄ oder -CO-R₅, bedeutet.

Für die obengenannten Ausführungsformen des erfindungsgemässen Verfahrens werden bevorzugt Verbindungen der Formel II, worin
Y für -CO-NHR₄ oder -CO-R₅;
R₄ für Wasserstoff oder Methyl;
R₅ für Methyl oder Phenyl;
R₁ und R₂ unabhängig voneinander für Wasserstoff, Chlor oder zusammen für Methylendioxy oder Difluormethylendioxy;
X für Cyano, -CO-R₃ oder -CO-OR₃; und
R₃ für Methyl stehen,
eingesetzt.

Durch das erfindungsgemässe Verfahren wird es möglich, Verbindungen der Formel I in guten Ausbeuten und auf einfache Weise herzustellen.

Ein besonderer Vorteil des erfindungsgemässen Verfahrens besteht darin, dass das als Zwischenprodukt hergestellte (p-Toluolsulfonyl)-methylisocyanid für die Umsetzung mit der Verbindung der Formel II nicht aus seinem Reaktionsmedium isoliert und gereinigt werden muss. Die bei der Isolierung und Reinigung von (p-Toluolsulfonyl)-methylisocyanid auftretenden Sicherheitsrisiken, hervorgerufen durch eine mögliche Detonation des getrockneten, kristallinen Produktes, werden bei erfindungsgemässen Verfahren vollständig vermieden. Neben der Vermeidung einer kostenintensiven wasserfreien Reaktionsführung ist ferner hervorzuheben, dass die durch das erfindungsgemässe Verfahren ermöglichte, erheblich vereinfachte Handhabbarkeit des (p-Toluolsulfonyl)-methylisocyanids einen geringeren sicherheitstechnischen Aufwand erfordert als bei den bisher bekannten Verfahren.

Ein weiterer Vorteil des erfindungsgemässen Verfahrens besteht darin, dass sich das Reaktionsgemisch nach Zugabe von Wasser in zwei flüssige Phasen trennt, von denen die organische Phase das gewünschte Zwischenprodukt und die wässrige Phase die Nebenprodukte enthält. Somit wird das Zwischenprodukt nach Trennung der beiden Phasen direkt in Form einer Lösung in dem verwendeten Lösungsmittel erhalten.

Das erfindungsgemässe Verfahren wird durch die folgenden Beispiele näher erläutert:

### Herstellungsbeispiele

### Beispiel H1: 4-Cyano-3-(2,3-dichlorphenyl)-pyrrol

a) Herstellung einer Lösung von (p-Toluolsulfonyl)-methylisocyanid:
   Zu einer Lösung bestehend aus 55,5 g (0,26 Mol) N-(p-Toluolsulfonyl)-methylformamid, 124 g Triäthylamin und 155 g 1,2-Dimethoxyäthan werden innerhalb von 2 bis 3 Stunden bei einer Temperatur von 0°C 41,5 g (0,27 Mol) Phosphoroxychlorid tropfenweise zugesetzt. Nach einstündigem Rühren bei 0°C wird die Reaktionsmischung unter Kühlung mit 220 ml Wasser vermischt. Man lässt die Innentemperatur auf +20°C ansteigen und trennt danach die untere wässrige Phase des entstandenen zweiphasigen Gemisches ab. Man erhält so 157 g einer Lösung von (p-Toluolsulfonyl)-methylisocyanid in 1,2-Dimethoxyäthan, die einen Produktgehalt von 23,2 Gew.% (72 % d.Th.) aufweist.
b) 157 g der gemäss a) hergestellten Lösung von (p-Toluolsulfonyl)-methylisocyanid versetzt man unter Rühren mit 48,3 g α-Cyano-2,3-dichlorzimtsäureamid. Nach Kühlen der Reaktionsmischung auf 0°C lässt man innerhalb von 2 Stunden unter Rühren 53,3 g (0,4 Mol) 30%iges, wässriges Natriumhydroxid zutropfen. Nach 2-stündigem Rühren bei 0°C werden 200 g Wasser zugegeben. Anschliessend wird die Reaktionslösung bei +70°C/200 mbar eingeengt und das Produkt nach Zugabe von 300 g Wasser bei Raumtemperatur abfiltriert. Nach Waschen des Filterrückstandes mit 80 g Toluol erhält man durch Trocknen im Vakuum 44,9 g 4-Cyano-3-(2,3-dichlorphenyl)-pyrrol (97 % d.Th.), Smp. 149°C.

### Beispiel H2: 4-Cyano-3-(2,3-methylendioxyphenyl)-pyrrol

96,9 g einer gemäss Beispiel H1a) hergestellten Lösung mit einem Gehalt von 23,2 Gew.% (p-Toluolsulfonyl)-methylisocyanid versetzt man bei 0°C unter Rühren mit 24,9 g α-Cyano-2,3-methylendioxyzimtsäureamid. Anschliessend lässt man innerhalb von 2 Stunden 41,4 g 30%ige methanolische Natriummethylatlösung zutropfen. Nach 30-minütigem Rühren wird 300 ml Wasser tropfenweise zugegeben und anschliessend abfiltriert. Nach Waschen des Filterrückstandes mit Toluol und Trocknen im Vakuum erhält man 21 g (86,5 % d.Th.) 4-Cyano-3-(2,3-methylendioxyphenyl)-pyrrol, Smp. 206-208°C.

### Beispiel H3: 4-Methylcarbonyl-3-(2,3-dichlorphenyl)-pyrrol

65 g eines gemäss Beispiel H1a) hergestellten Lösung mit einem Gehalt von 23,2 Gew.% (p-Toluolsulfonyl)-methylisocyanid versetzt man bei einer Temperatur von 0°C unter Rühren mit 20 g 1,1-Di-(methylcarbonyl)-2-(2,3-dichlorphenyl)-äthen. Anschliessend lässt man innerhalb von 3 Stunden 28,1 g 30%ige methanolische Natriummethylatlösung zutropfen. Nach 60-minütigem Rühren bei einer Temperatur von +10°C wird 170 ml Wasser tropfenweise zugegeben und anschliessend abfiltriert. Nach Umkristallisation des Filterrückstandes in einem Methanol/Aethanol-Gemisch (1:1) und Trocknen im Vakuum erhält man 8,3 g (50 % d.Th.) 4-Methylcarbonyl-3-(2,3-dichlorphenyl)-pyrrol, Smp. 192-194°C.

### Beispiel H4: 4-Cyano-3-(4-chlorphenyl)-pyrrol

117,4 g einer gemäss Beispiel H1a) hergestellten Lösung mit einem Gehalt von 23,2 Gew.% an (p-Toluolsulfonyl)-methylisocyanid versetzt man bei einer Temperatur von 0°C unter Rühren mit 28,8 g α-Cyano-4-chlorzimtsäureamid. Anschliessend lässt man bei einer Temperatur von +5°C innerhalb von 3 Stunden 50,4 g 30%ige methanolische Natriummethylatlösung zutropfen. Nach 2-stündigem Rühren wird 70 ml Wasser zugegeben. Anschliessend wird das Lösungsmittelgemisch im Vakuum bei 50°C um 50 ml eingeengt. Nach Zugabe von weiteren 140 ml Wasser wird die Reaktionsmischung filtriert und der Filterrückstand mit 30 ml Toluol gewaschen. Nach Umkristallisation des getrockneten Filterrückstandes aus Toluol erhält man 25,4 g (89,5 % d.Th.) 4-Cyano-3-(4-chlorphenyl)-pyrrol, Smp. 147-148°C.

### Beispiel H5: 4-Methoxycarbonyl-3-(2,3-dichlorphenyl)-pyrrol

65 g einer gemäss Beispiel H1a) hergestellten Lösung mit einem Gehalt von 22 Gew.% (p-Toluolsulfonyl)-methylisocyanid versetzt man bei 0°C unter Rühren mit 20 g α-Methylcarbonyl-2,3-dichlorzimtsäuremethylester Anschliessend lässt man innerhalb von 2 Stunden 26,3 g 30%ige methanolische Natriummethylatlösung zutropfen. Nach 2-stündigem Rühren wird 35 ml Wasser tropfenweise zugegeben. Anschliessend wird die Reaktionsmischung bei +50°C im Vakuum eingeengt. Nach Zugabe von 70 ml Wasser und erneutem Abdampfen im Vakuum wird die Reaktionsmischung filtriert. Nach Waschen des Filterrückstandes mit Toluol und Umkristallisieren aus Ethanol erhält man 10,8 g (58 % d.Th.) 4-Methoxycarbonyl-3-(2,3-dichlorphenyl)-pyrrol, Smp. 200-201°C.

### Beispiel H6: 4-Cyano-3-(2,3-dichlorphenyl)-pyrrol

a) Herstellung einer Lösung von (p-Toluolsulfonyl)-methylisocyanid
   Zu einer Lösung bestehend aus 59,6 g (0,28 Mol) N-(p-Toluolsulfonyl)-methylformamid, 134 g Triäthylamin und 168 g Tetrahydrofuran werden innerhalb von 2 Stunden bei einer Temperatur von -5°C 44,5 g Phosphoroxytrichlorid tropfenweise zugegeben. Nach einstündigem Rühren bei 0°C wird die Reaktionsmischung tropfenweise mit 300 ml Wasser vermischt. Während dieser Massnahme lässt man die Temperatur auf +20°C ansteigen. Nach Abt rennen der unteren wässrigen Phase des entstandenen zweiphasigen Gemisches erhält man 198 g einer Lösung von (p-Toluolsulfonyl)-methylisocyanid in Tetrahydrofuran, die 38 g des Produktes enthält.
b) 198 g der gemäss a) hergestellten Lösung von (p-Toluolsulfonyl)-methylisocyanid versetzt man unter Rühren mit 46,9 g α-Cyano-2,3-dichlorzimtsäureamid. Nach Kühlen der Reaktionsmischung auf +5°C lässt man innerhalb von 90 Minuten unter Rühren 43,6 g 50%ige wässrige Kaliumhydroxidlösung zutropfen. Nach 2-stündigem Rühren bei 20°C werden 80 ml Wasser zugegeben. Anschliessend werden 50 ml des Lösungsmittelgemisches abgedampft. Nach Zugabe von weiteren 150 ml Wasser und erneutem Abdampfen wird die Reaktionsmischung filtriert. Nach Waschen des Filterrückstandes mit Toluol und Umkristallisieren aus Methanol erhält man 34,2 g (85 % d.Th.) 4-Cyano-3-(2,3-dichlorphenyl)-pyrrol, Smp. 146-148°C.

### Beispiel H7: Herstellung von 4-Cyano-3-(2,3-dichlorphenyl)-pyrrol

a) Herstellung einer Lösung aus (p-Toluolsulfonyl)-methylisocyanid:
   Zu einer Suspension bestehend aus 43 g (0,2 Mol) N-(p-Toluolsulfonyl)-methylformamid, 96,1 g Triäthylamin und 145 g Essigsäureäthylester werden innerhalb von 2 Stunden bei einer Temperatur von 0 bis +5°C 31,5 g Phosphoroxychlorid tropfweise zugesetzt. Nach 30-minütigem Rühren bei 0 bis +5°C wird die Reaktionsmischung mit 150 ml Wasser versetzt und anschliessend weitere 10 Minuten bei +20°C gerührt. Nach Abtrennung der wässrigen Phase wird die organische Phase mit 100 ml wässriger 1N Natriumhydroxidlösung versetzt. Nach 10 minütigem Rühren wird die wässrige Phase abgetrennt und die organische Phase mit 50 ml Wasser gewaschen. Man erhält 150 g einer Lösung von (p-Toluolsulfonyl)-methylisocyanid in Essigsäureäthylester, die einen Produktgehalt von 31 g aufweist.
b) 150 g der gemäss a) hergestellten Lösung von (p-Toluolsulfonyl)-methylisocyanid versetzt man unter Rühren mit 38,6 g α-Cyano-2,3-dichlorzimtsäureamid. Anschliessend lässt man bei einer Temperatur von 0°C innerhalb von 3 Stunden 63,6 g einer 30 %-igen methanolischen Natriummethylatlösung zutropfen. Nach Zugabe von 100 ml Wasser wird die Reaktionsmischung für weitere 15 Minuten gerührt und die wässrige Phase anschliessend abgetrennt. Nach erneuter Zugabe von Wasser wird das Lösungsmittel bei reduziertem Druck bei einer Temperatur von +50 bis +60°C vollständig entfernt. Die so erhaltene Suspension wird auf Raumtemperatur gekühlt und anschliessend filtiriert. Nach Waschen des Filterrückstandes mit 200 ml Toluol und anschliessender Trocknung erhält man 31 g (82 % d. Th.) 4-Cyano-3-(2,3-dichlorphenyl)-pyrrol mit einem Schmelzpunkt von +148°C.

### Beispiel H8: Herstellung von 4-Cyano-3-(2,3-difluormethylendioxyphenyl)-pyrrol

a) Herstellung einer Lösung von (p-Toluolsulfonyl)-methylisocyanid
   Zu einer Suspension bestehend aus 42,6 g (0,2 Mol) N-(p-Toluolsulfonyl)-methylformamid, 96 g (0,95 Mol) Triäthylamin und 160 g Essigsäureäthylester werden innerhalb von 1-2 Stunden bei einer Temperatur von 0 bis +5°C 31,8 g (0,208 Mol) Phosphoroxytrichlorid tropfenweise zudosiert. Nach einstündigem Rühren bei 0 bis +5°C wird die Reaktionsmischung langsam mit 150 ml Wasser versetzt; dabei lässt man die Temperatur auf +20°C ansteigen. Die untere wässrige Phase wird abgetrennt und die organische Phase bei +15 bis +20°C mit 100 ml Natriumcarbonatlösung 10 % ausgewaschen. Nach Abtrennen der unteren wässrigen Phase erhält man 210 g einer Lösung von (p-Toluolsulfonyl)-methylisocyanid in Essigsäureäthylester, die 33,2 g (0,17 Mol) des Produktes (=85 % d. Th.) enthält.
b) Zu 210 g der gemäss a) hergestellten Lösung von (p-Toluolsulfonyl)-methylisocyanid gibt man 42,8 g (0,17 Mol) α-Cyano-2,3-difluormethylendioxyzimtsäureamid. Zu dieser Suspension dosiert man innerhalb von 1 Stunde unter Rühren bei +5 bis +10°C 45,8 g (0,204 Mol) 25 %-ige methanolische Kaliumhydroxidlösung. Nach 1-stündigem Rühren bei +20°C kann nach folgenden Methoden aufgearbeitet werden:
   Methode 1: Bei +65 bis + 70°C werden ca. 120 ml Essigsäureäthylester/Methanol-Gemisch abdestilliert. Nach Zugabe von 300 ml Methanol und erneutem Abdampfen von ca. 170 ml Lösungsmittel wird die Reaktionsmischung mit 90 ml Wasser versetzt und filtriert. Nach Waschen des Filterrückstandes mit Methanol/Wasser-Gemisch erhält man 35,8 g (85 % d. Th.) 4-Cyano-3-(2,3-difluormethylendioxyphenyl)-pyrrol mit einem Schmelzpunkt von +190° bis +195°C.
   Methode 2: Bei +65 bis +70°C werden ca. 120 ml Essigsäureäthylester/Methanol-Gemisch abdestilliert. Nach Zugabe von 120 ml Wasser und erneutem Abdampfen wird die Reaktionsmischung mit 120 ml Methanol versetzt und filtriert. Nach Waschen des Filterrückstandes mit Methanol/Wasser-Gemisch erhält man 34,3 g (83 % d. Th.) 4-Cyano-3-(2,3-difluormethylendioxyphenyl)-pyrrol mit einem Schmelzpunkt von +190 bis +195°C.
   Methode 3: Nach Zugabe von 200 ml Wasser und Abtrennen der unteren wässrigen Phase werden 120 ml Essigsäureäthylester abdestilliert und der Rückstand mit 300 ml Methanol versetzt. Nachdem erneutem Abdampfen von ca. 170 ml Lösungsmittel und Zugabe von 90 ml Wasser wird die Reaktionsmischung filtriert. Nach Waschen des Filterrückstandes mit Methanol/Wasser-Gemisch erhält man 34,3 g (83 % d. Th.) 4-Cyano-3-(2,3-difluormethylendioxyphenyl)-pyrrol mit einem Schmelzpunkt von +190 bis +195°C.

## Patentansprüche

1. Verfahren zur Herstellung von 3-Phenylpyrrolderivaten der Formel I 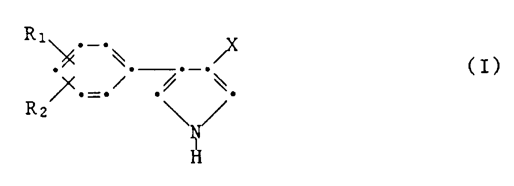 worin X für Cyano, -CO-R₃, -CO-OR₃ oder 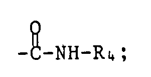 R₁ und R₂ unabhängig voneinander für Wasserstoff, C₁-C₆-Alkyl, C₁-C₆-Alkoxy, C₁-C₆-Alkylthio, Nitro, Cyano, Halogen oder C₁-C₆-Halogenalkyl, oder
R₁ und R₂ zusammen für Methylendioxy oder Difluormethylendioxy stehen;
R₃ C₁-C₆-Alkyl, C₁-C₆-Halogenalkyl, Phenyl oder Benzyl oder durch Halogen, Methyl, Methoxy oder Methylthio substituiertes Phenyl oder Benzyl bedeutet und
R₄ für Wasserstoff, C₁-C₆-Alkyl, C₁-C₆-Halogenalkyl, Phenyl oder Benzyl oder durch Halogen, Methyl, Methoxy oder Methylthio substituiertes Phenyl oder Benzyl steht,
dadurch gekennzeichnet, dass man
a) N-(p-Toluolsulfonyl)-methylformamid in einem inerten Lösungsmittel in Gegenwart einer organischen Base mit Phosphoroxychlorid umsetzt, die Reaktionslösung mit Wasser vermischt, die wässrige Phase abtrennt, und
b) die (p-Toluolsulfonyl)-methylisocyanid enthaltende organische Phase unmittelbar mit einer Verbindung der Formel II 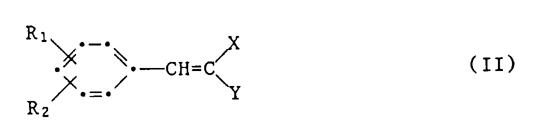 worin X, R₁ und R₂ die unter Formel I angegebene Bedeutung haben;
Y für -CO-NHR₄, -CO-R₅ oder -S-R₅ steht;
R₅ für C₁-C₆-Alkyl, C₁-C₆-Halogenalkyl, Phenyl oder Benzyl oder durch Halogen, Methyl, Methoxy oder Methylthio substituiertes Phenyl oder Benzyl steht; und
R₄ die unter Formel I angegebenen Bedeutung hat, in Gegenwart einer Base umsetzt.

2. Verfahren nach Anspruch 1, dadurch gekennzeichnet, dass man
a) die Verbindung der Formel II gemeinsam mit der (p-Toluolsulfonyl)-methylisocyanidlösung vorlegt und
b) die Base zudosiert.

3. Verfahren nach Anspruch 1, dadurch gekennzeichnet, dass man bei der Umsetzung von (p-Toluolsulfonyl)-methylisocyanid mit der Verbindung der Formel II die Base in einer Menge von 2 bis 3 Mol pro Mol Verbindung der Formel II einsetzt.

4. Verfahren nach Anspruch 1, dadurch gekennzeichnet, dass R₁ und R₂ unabhängig voneinander für Wasserstoff, Halogen, Methoxy oder Methylthio oder zusammen für Methylendioxy oder Difluormethylendioxy stehen; R₃ und R₅ unabhängig voneinander C₁-C₄-Alkyl oder C₁-C₄-Halogenalkyl bedeuten; R₄ für Wasserstoff, C₁-C₄-Alkyl oder C₁-C₄-Halogenalkyl steht und Y -CO-NHR₄ oder -CO-R₅ bedeutet.

5. Verfahren nach Anspruch 1, dadurch gekennzeichnet, dass man die Verbindung der Formel II in einem Ueberschuss von 10 bis 20 Mol% in bezug auf die Menge an (p-Toluolsulfonyl)-methylisocyanid einsetzt.

6. Verfahren nach Anspruch 1, dadurch gekennzeichnet, dass man das (p-Toluolsulfonyl)-methylisocyanid und die Verbindung der Formel II in äquimolaren Mengen einsetzt.

7. Verfahren nach Anspruch 1, dadurch gekennzeichnet, dass man als inertes Lösungsmittel eine Verbindung oder Gemisch von Verbindungen aus der Gruppe der offenkettigen oder zyklischen Aether, Chloralkane, Ketone oder aus der Gruppe der Alkylester niederer Carbonsäuren verwendet.

8. Verfahren nach Anspruch 1, dadurch gekennzeichnet, dass man als inertes Lösungsmittel eine Verbindung oder Gemische von Verbindungen aus der Gruppe der offenkettigen oder zyklischen Aether oder aus der Gruppe der Chloralkane verwendet.

9. Verfahren nach Anspruch 1, dadurch gekennzeichnet, dass man bei der Umsetzung von N-(p-Toluolsulfonyl)-methylformamid mit Phosphoroxychlorid ein tertiäres Amin als Base verwendet.

10. Verfahren nach Anspruch 1, dadurch gekennzeichnet, dass man bei der Umsetzung der Lösung von (p-Toluolsulfonyl)-methylisocyanid mit der Verbindung der Formel II als Base ein Oxid, Hydrid, Hydroxid, Carbonat, Carbonsäuresalz oder Alkoholat eines Alkali- oder Erdalkalimetalls, ein Trialkylamin oder eine Pyridinbase verwendet.

11. Verfahren nach Anspruch 1, dadurch gekennzeichnet, dass man die Umsetzung der Verbindung der Formel II mit p-(Toluolsulfonyl)-methylisocyanid bei einer Temperatur von -25°C bis +25°C durchführt.

12. Verfahren nach Anspruch 1, dadurch gekennzeichnet, dass man die Umsetzung der Verbindung der Formel II mit (p-Toluolsulfonyl)-methylisocyanid bei einer Temperatur von -10°C bis +10°C durchführt.

13. Verfahren nach Anspruch 10, dadurch gekennzeichnet, dass man als Base Natriummethylat, Natriumäthylat, wässriges Natriumhydroxid, wässriges Kaliumhydroxid, Natriumcarbonat oder Kaliumcarbonat verwendet.

14. Verfahren nach Anspruch 10, dadurch gekennzeichnet, dass man die Base in einer Menge von 2 bis 2,4 Mol pro Mol Verbindung der Formel II einsetzt.

15. Verfahren nach Anspruch 9, dadurch gekennzeichnet, dass man als Base Triäthylamin verwendet.

16. Verfahren nach Anspruch 1, dadurch gekennzeichnet, dass man als inertes Lösungsmittel Dimethoxymethan, 1,2-Dimethoxyäthan, Diäthyläther, Diisopropyläther, Tetrahydrofuran, Dioxan, Methylenchlorid, Chloroform Tetrachlorkohlenstoff oder Essigsäureäthylester verwendet.

17. Verfahren nach Anspruch 1, dadurch gekennzeichnet, dass man als inertes Lösungsmittel Dimethoxymethan, 1,2-Dimethoxyäthan, Diäthyläther, Diisopropyläther, Tetrahydrofuran, Dioxan, Methylenchlorid, Chloroform oder Tetrachlorkohlenstoff verwendet.

18. Verfahren nach Anspruch 1, dadurch gekennzeichnet, dass man als inertes Lösungsmittel Dimethoxymethan, 1,2-Dimethoxyäthan, Tetrahydrofuran, Dioxan oder Essigsäureäthylester verwendet.

19. Verfahren nach Anspruch 1, dadurch gekennzeichnet, dass man als inertes Lösungsmittel Dimethoxymethan, 1,2-Dimethoxyäthan, Tetrahydrofuran oder Dioxan verwendet.

20. Verfahren nach Anspruch 1, dadurch gekennzeichnet, dass man als inertes Lösungsmittel 1,2-Dimethoxyäthan verwendet und die nach Umsetzung von N-(p-Toluolsulfonyl)-methylformamid mit Phosphoroxychlorid in Gegenwart von Triäthylamin erhaltene Reaktionsmischung mit dem 0,5- bis 0,7-fachen Volumen an Wasser vermischt.

21. Verfahren nach Anspruch 1, dadurch gekennzeichnet, dass man als inertes Lösungsmittel Dioxan verwendet und die nach Umsetzung von N-(p-Toluolsulfonyl)-methylformamid mit Phosphoroxychlorid erhaltene Reaktionsmischung mit dem 0,2- bis 0,35-fachen Volumen an Wasser vermischt.

22. Verfahren nach Anspruch 1, dadurch gekennzeichnet, dass man als inertes Lösungsmittel Tetrahydrofuran verwendet und die nach Umsetzung von N-(p-Toluolsulfonyl)-methylformamid mit Phosphoroxychlorid erhaltene Reaktionsmischung mit dem 0,3- bis 0,8-fachen Volumen an Wasser vermischt.

23. Verfahren nach Anspruch 1, dadurch gekennzeichnet, dass man als inertes Lösungsmittel Dimethoxymethan verwendet und die nach Umsetzung von N-(p-Toluolsulfonyl)-methylformamid mit Phosphoroxychlorid erhaltene Reaktionsmischung mit dem 0,3- bis 1,2-fachen Volumen an Wasser vermischt.

24. Verfahren nach Anspruch 1, dadurch gekennzeichnet, dass X für Cyano, -CO-OR₃ oder -CO-R₃ steht; R₁ und R₂ unabhängig voneinander Wasserstoff, Chlor oder zusammen Methylendioxy oder Difluormethylendioxy bedeuten; R₃ für Methyl steht; Y -CO-NHR₄ oder -CO-R₅ bedeutet; R₄ für Wasserstoff oder Methyl, und R₅ für Methyl oder Phenyl stehen.

25. Verfahren nach Anspruch 1, dadurch gekennzeichnet, dass man
a) die Umsetzung von N-(p-Toluolsulfonyl)-methylformamid mit Phosphoroxychlorid in 1,2-Dimethoxyäthan in Gegenwart von Triäthylamin durchführt, die Reaktionsmischung mit dem 0,55- bis 0,6-fachen Volumen an Wasser vermischt, die wässrige Phase aus dem so erhaltenen zweiphasigen Reaktionsgemisch abtrennt, und
b) die (p-Toluolsulfonyl)-methylisocyanid enthaltende organische Phase unmittelbar mit einer äquimolaren Menge an Verbindung der Formel II in bezug auf die Menge an (p-Toluolsulfonyl)-methylisocyanid in Gegenwart von 2 bis 2,4 Mol wässrigem Natriumhydroxid in bezug auf die Verbindung der Formel II bei einer Temperatur von -10°C bis +10°C umsetzt.

26. Verfahren nach Anspruch 22, dadurch gekennzeichnet, dass man
a) die Verbindung der Formel II gemeinsam mit der (p-Toluolsulfonyl)-methylisocyanidlösung vorlegt und
b) das wässrige Natriumhydroxid zudosiert.

## Claims

1. A process for the preparation of a 3-phenylpyrrole derivative of formula I 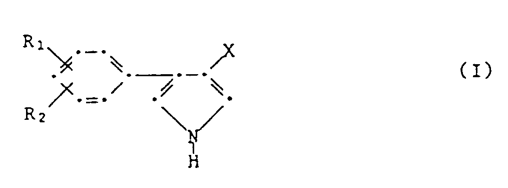 in which
X is cyano, -CO-R₃, -CO-OR₃ or 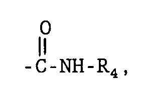 R₁ and R₂ are each independently of the other hydrogen, C₁-C₆alkyl, C₁-C₆alkoxy, C₁-C₆alkylthio, nitro, cyano, halogen or C₁-C₆haloalkyl, or
R₁ and R₂, when taken together, are methylenedioxy or difluoromethylenedioxy,
R₃ is C₁-C₆alkyl, C₁-C₆haloalkyl, phenyl or benzyl, or phenyl or benzyl which are each substituted by halogen, methyl, methoxy or methylthio,
R₄ is hydrogen, C₁-C₆alkyl, C₁-C₆haloalkyl, phenyl or benzyl, or phenyl or benzyl which are each substituted by halogen, methyl, methoxy or methylthio, which process comprises
a) reacting N-(p-toluenesulfonyl)methylformamide, in an inert solvent and in the presence of an organic base, with phosphorus oxychloride, mixing the reaction solution with water, separating off the aqueous phase, and
b) reacting the organic phase containing (p-toluenesulfonyl)methyl isocyanide direct with a compound of formula II 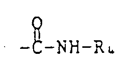 in which X, R₁ and R₂ are as defined for formula I, Y is -CO-NHR₄, -CO-R₅ or -S-R₅, R₅ is C₁-C₆alkyl, C₁-C₆haloalkyl, phenyl or benzyl, or phenyl or benzyl which are each substituted by halogen, methyl, methoxy or methylthio, and R₄ is as defined for formula I, in the presence of a base.

2. A process according to claim 1, which comprises
a) adding the compound of formula II to the solution of (p-toluenesulfonyl)methyl isocyanide, and
b) adding the base to the reaction mixture.

3. A process according to claim 1, which comprises adding the base in an amount of 2 to 3 mol per mol of compound of formula II in the reaction of (p-toluenesulfonyl)methyl isocyanide with the compound of formula II.

4. A process according to claim 1, wherein R₁ and R₂ are each independently of the other hydrogen, halogen, methoxy or methylthio or, when taken together, are methylenedioxy or difluoromethylenedioxy, R₃ and R₅ are independently of each other C₁-C₄alkyl or C₁-C₄haloalkyl, R₄ is hydrogen, C₁-C₄alkyl or C₁-C₄haloalkyl, and Y is -CO-NHR₄ or -CO-R₅.

5. A process according to claim 1, wherein the compound of formula II is used in an excess of 10 to 20 mol%, based on the amount of (p-toluenesulfonyl)methyl isocyanide.

6. A process according to claim 1, wherein (p-toluenesulfonyl)methyl isocyanide and the compound of formula II are used in equimolar amounts.

7. A process according to claim 1, wherein the inert solvent is a compound or a mixture of compounds selected from the group of the open chain or cyclic ethers, chloroalkanes, ketones or from the group of the alkyl esters of lower carboxylic acids.

8. A process according to claim 1, wherein the inert solvent is a compound or a mixture of compounds selected from the group of the open-chain or cyclic ethers or from the group of the chloroalkanes.

9. A process according to claim 1, wherein a tertiary amine is used as base in the reaction of N-(p-toluenesulfonyl)methylformamide with phosphorus oxychloride.

10. A process according to claim 1, wherein an oxide, hydride, hydroxide, carbonate, carboxylic acid salt or alcoholate of an alkali metal or alkaline earth metal, a trialkylamine or a pyridine base is used as base for the reaction of the solution of (p-toluenesulfonyl)methyl isocyanide with the compound of formula II.

11. A process according to claim 1, wherein the reaction of the compound of formula II with (p-toluenesulfonyl)methyl isocyanide is carried out in the temperature range from -25°C to +25°C.

12. A process according to claim 1, wherein the reaction of the compound of formula II with (p-toluenesulfonyl)methyl isocyanide is carried out in the temperature range from -10°C to +10°C.

13. A process according to claim 10, wherein the base is sodium methylate, sodium ethylate, aqueous sodium hydroxide, aqueous potassium hydroxide, sodium carbonate or potassium carbonate.

14. A process according to claim 10, wherein the base is used in an amount of 2 to 2.4 mol per mol of compound of formula II.

15. A process according to claim 9, wherein the base is triethylamine.

16. A process according to claim 1, wherein the inert solvent is dimethoxymethane, 1,2-dimethoxyethane, diethyl ether, diisopropyl ether, tetrahydrofuran, dioxane, methylene chloride, chloroform, carbon tetrachloride or ethyl acetate.

17. A process according to claim 1, wherein the inert solvent is dimethoxymethane, 1,2-dimethoxyethane, diethyl ether, diisopropyl ether, tetrahydrofuran, dioxane, methylene chloride, chloroform, or carbon tetrachloride.

18. A process according to claim 1, wherein the inert solvent is dimethoxymethane, 1,2-dimethoxyethane, tetrahydrofuran, dioxane or ethyl acetate.

19. A process according to claim 1, wherein the inert solvent is dimethoxymethane, 1,2-dimethoxyethane, tetrahydrofuran, or dioxane.

20. A process according to claim 1, wherein the inert solvent is 1,2-dimethoxyethane and the reaction mixture obtained by reaction of N-(p-toluenesulfonyl)methylformamide with phosphorus oxychloride in the presence of triethylamine is mixed with the 0.5- to 0.7-fold volume of water.

21. A process according to claim 1, wherein the inert solvent is dioxane and the reaction mixture obtained by reaction of N-(p-toluenesulfonyl)methylformamide with phosphorus oxychloride is mixed with the 0.2- to 0.35-fold volume of water.

22. A process according to claim 1, wherein the inert solvent is tetrahydrofuran and the reaction mixture obtained by reaction of N-(p-toluenesulfonyl)methylformamide with phosphorus oxychloride is mixed with the 0.3- to 0.8-fold volume of water.

23. A process according to claim 1, wherein the inert solvent is dimethoxymethane and the reaction mixture obtained by reaction of N-(p-toluenesulfonyl)methylformamide with phosphorus oxychloride is mixed with the 0.3- to 1.2-fold volume of water.

24. A process according to claim 1, wherein X is cyano, -CO-OR₃ or -CO-R₃, R₁ and R₂ are each independently of the other hydrogen, chlorine or, when taken together, are methylenedioxy or difluoromethylenedioxy, R₃ is methyl, Y is -CO-NHR₄ or -CO-R₅, R₄ is hydrogen or methyl, and R₅ is methyl or phenyl.

25. A process according to claim 1, which comprises
a) carrying out the reaction of N-(p-toluenesulfonyl)methylformamide with phosphorus oxychloride in 1,2-dimethoxyethane in the presence of triethylamine, mixing the reaction mixture with the 0.55- to 0.6-fold volume of water, separating off the aqueous phase from the resultant two-phase reaction mixture, and
b) reacting the organic phase containing (p-toluenesulfonyl)methyl isocyanide with the equimolar amount of compound of formula II, based on the amount of (p-toluenesulfonyl)methyl isocyanide, in the presence of 2 to 2.4 mol of aqueous sodium hydroxide, based on the compound of formula II, in the temperature range from -10°C to +10°C.

26. A process according to claim 22, which comprises
a) adding the compound of formula II to the solution of (p-toluenesulfonyl)-methyl isocyanide, and
b) adding aqueous sodium hydroxide to the reaction mixture.

## Revendications

1. Procédé pour la préparation des dérivés de 3-phénylpyrroles de formule I 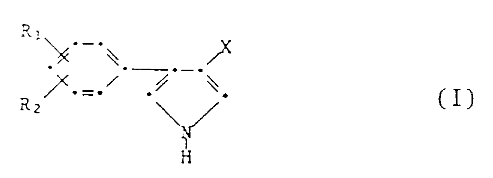 où X représente le cyano, -CO-R₃, -CO-OR₃ ou 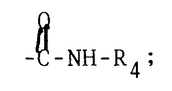 R₁ et R₂ représentent, indépendamment l'un de l'autre, l'hydrogène, un alkyle en C₁-C₆, un alcoxy en C₁-C₆, un alkyle en C₁-C₆thio, le nitro, le cyano, un halogène ou un halogénoalkyle en C₁-C₆ ou
R₁ et R₂ représentent ensemble le méthylènedioxy ou le difluorométhylènedioxy;
R₃ représente un alkyle en C₁-C₆, un halogénoalkyle en C₁-C₆, le phényle ou le benzyle ou un phényle ou un benzyle substitué par un halogène, le méthyle, le méthoxy ou le méthylthio;
R₄ représente l'hydrogène, un alkyle en C₁-C₆, un halogénoalkyle en C₁-C₆, le phényle ou le benzyle ou un phényle ou un benzyle substitué par un halogène, le méthyle, le méthoxy ou le méthylthio,
caractérisé
a) en ce que l'on fait réagir le N-(p-toluènesulfonyl)-méthylformamide dans un solvant inerte en présence d'une base organique avec de l'oxychlorure de phosphore, en ce que l'on mélange la solution réactionnelle avec de l'eau, en ce que l'on sépare la phase aqueuse, et
b) en ce que l'on fait immédiatement réagir la phase organique contenant le (p-toluènesulfonyl)-méthylisocyanure avec un composé de formule II 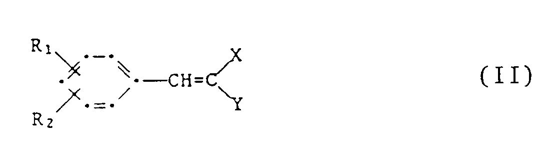 où X, R₁ et R₂ ont les significations données pour la formule I;
Y représente -CO-NHR₄, -CO-R₅ ou -S-R₅;
R₅ représente un alkyle en C₁-C₆, un halogénoalkyle en C₁-C₆, le phényle ou le benzyle ou un phényle ou un benzyle substitué par un halogène, le méthyle, le méthoxy ou le méthylthio; et
R₄ a la signification donnée pour la formule I, en présence d'une base.

2. Procédé selon la revendication 1, caractérisé
a) en ce que l'on introduit préalablement le composé de formule II et la solution de (p-toluènesulfonyl)-méthylisocyanure et
b) en ce que l'on ajoute la base.

3. Procédé selon la revendication 1, caractérisé en ce que l'on utilise pour la réaction du (p-toluènesulfonyl)-méthylisocyanure avec le composé de formule II la base en une quantité allant de 2 à 3 moles par mole de composé de formule II.

4. Procédé selon la revendication 1, caractérisé en ce que R₁ et R₂ représentent, indépendamment l'un de l'autre, l'hydrogène, un halogène, le méthoxy ou le méthylthio, ou représentent ensemble le méthylènedioxy ou le difluorométhylènedioxy; R₃ et R₅ représentent, indépendamment l'un de l'autre, un alkyle en C₁-C₄ ou un halogénoalkyle en C₁-C₄; R₄ représente l'hydrogène, un alkyle en C₁-C₄ ou un halogénoalkyle en C₁-C₄ et Y représente -CO-NHR₄ ou -CO-R₅.

5. Procédé selon la revendication 1, caractérisé en ce que l'on utilise le composé de formule II avec un excès allant de 10 à 20% mole rapporté à la quantité de (p-toluènesulfonyl)-méthylisocyanure.

6. Procédé selon la revendication 1, caractérisé en ce que l'on utilise le (p-toluènesulfonyl)-méthylisocyanure et le composé de formule II en quantités équimolaires.

7. Procédé selon la revendication 1, caractérisé en ce que l'on utilise, comme solvant inerte, un composé ou un mélange de composés provenant du groupe des éthers à chaîne ouverte ou des éthers cycliques, des chloroalcanes, des cétones ou provenant du groupe des esters alkyliques des acides carboxyliques inférieurs.

8. Procédé selon la revendication 1, caractérisé en ce que l'on utilise, comme solvant inerte, un composé ou des mélanges de composés provenant du groupe des éthers à chaîne ouverte ou des éthers cycliques ou provenant du groupe des chloroalcanes.

9. Procédé selon la revendication 1, caractérisé en ce que l'on utilise pour la réaction du N-(p-toluènesulfonyl)-méthylformamide avec l'oxychlorure de phosphore, une amine tertiaire comme base.

10. Procédé selon la revendication 1, caractérisé en ce que l'on utilise pour la réaction de la solution de (p-toluènesulfonyl)-méthylisocyanure avec le composé de formule II, comme base, un oxyde, un hydrure, un hydroxyde, un carbonate, un sel d'acide carboxylique ou un alcoolate d'un métal alcalin ou d'un métal alcalino-terreux, une trialkylamine ou une base pyridique.

11. Procédé selon la revendication 1, caractérisé en ce que l'on effectue la réaction du composé de formule II avec le (p-toluènesulfonyl)-méthylisocyanure à une température allant de -25°C à +25 C.

12. Procédé selon la revendication 1, caractérisé en ce que l'on effectue la réaction du composé de formule II avec le (p-toluènesulfonyl)-méthylisocyanure à une température allant de -10°C à +10°C.

13. Procédé selon la revendication 10, caractérisé en ce que l'on utilise, comme base, le méthylate de sodium, l'éthylate de sodium, l'hydroxyde de sodium en solution aqueuse, l'hydroxyde de potassium en solution aqueuse, le carbonate de sodium ou le carbonate de potassium.

14. Procédé selon la revendication 10, caractérisé en ce que l'on utilise la base en une quantité allant de 2 à 2,4 moles par mole de composé de formule II.

15. Procédé selon la revendication 9, caractérisé en ce que l'on utilise, comme base, la triéthylamine.

16. Procédé selon la revendication 1, caractérisé en ce que l'on utilise, comme solvant inerte, le diméthoxyméthane, le 1,2-diméthoxyéthane, l'éther diéthylique, l'éther diisopropylique, le tétrahydrofuranne, le dioxane, le chlorure de méthylène, le chloroforme, le tétrachlorure de carbone ou l'acétate d'éthyle.

17. Procédé selon la revendication 1, caractérisé en ce que l'on utilise, comme solvant inerte, le diméthoxyméthane, le 1,2-diméthoxyéthane, l'éther diéthylique, l'éther diisopropylique, le tétrahydrofuranne, le dioxane, le chlorure de méthylène, le chloroforme ou le tétrachlorure de carbone.

18. Procédé selon la revendication 1, caractérisé en ce que l'on utilise, comme solvant inerte, le diméthoxyméthane, le 1,2-diméthoxyéthane, le tétrahydrofuranne, le dioxane ou l'acétate d'éthyle.

19. Procédé selon la revendication 1, caractérisé en ce que l'on utilise, comme solvant inerte, le diméthoxyméthane, le 1,2-diméthoxyéthane, le tétrahydrofuranne ou le dioxane.

20. Procédé selon la revendication 1, caractérisé en ce que l'on utilise, comme solvant inerte, le 1,2-diméthoxyéthane et en ce que l'on mélange le mélange réactionnel, obtenu après réaction du N-(p-toluènesulfonyl)-méthylformamide avec l'oxychlorure de phosphore en présence de triéthylamine, avec 0,5 à 0,7 fois son volume d'eau.

21. Procédé selon la revendication 1, caractérisé en ce que l'on utilise, comme solvant inerte, le dioxane et en ce que l'on mélange le mélange réactionnel, obtenu après réaction du N-(p-toluènesulfonyl)-méthylformamide avec l'oxychlorure de phosphore, avec 0,2 à 0,35 fois son volume d'eau.

22. Procédé selon la revendication 1, caractérisé en ce que l'on utilise, comme solvant inerte, le tétrahydrofuranne et en ce que l'on mélange le mélange réactionnel, obtenu après réaction du N-(p-toluènesulfonyl)-méthylformamide avec l'oxychlorure de phosphore, avec 0,3 à 0,8 fois son volume d'eau.

23. Procédé selon la revendication 1, caractérisé en ce que l'on utilise, comme solvant inerte, le diméthoxyméthane et en ce que l'on mélange le mélange réactionnel, obtenu après réaction du N-(p-toluènesulfonyl)-méthylformamide avec l'oxychlorure de phosphore, avec 0,3 à 1,2 fois son volume d'eau.

24. Procédé selon la revendication 1, caractérisé en ce que X représente le cyano, -CO-OR₃ ou -CO-R₃;
R₁ et R₂ représentent, indépendamment l'un de l'autre, l'hydrogène, le chlore ou représentent ensemble le méthylènedioxy ou le difluorométhylènedioxy;
R₃ représente le méthyle; Y représente -CO-NHR₄ ou -CO-R₃; R₄ représente l'hydrogène ou le méthyle et R₅ représente le méthyle ou le phényle.

25. Procédé selon la revendication 1, caractérisé
a) en ce que l'on effectue la réaction du N-(p-toluènesulfonyl)-méthylformamide avec l'oxychlorure de phosphore dans du 1,2-diméthoxyéthane en présence de triéthylamine, en ce que l'on mélange le mélange réactionnel avec 0,55 à 0,6 fois son volume d'eau, en ce que l'on sépare la phase aqueuse du mélange réactionnel à deux phases ainsi obtenu et
b) en ce que l'on fait immédiatement réagir la phase organique contenant le (p-toluènesulfonyl)-méthylisocyanure avec une quantité équimolaire du composé de formule II rapportée à la quantité de (p-toluènesulfonyl)-méthylisocyanure, en présence de 2 à 2,4 moles d'hydroxyde de sodium en solution aqueuse, rapporté au composé de formule II, à une température allant de -10°C à +10°C.

26. Procédé selon la revendication 22, caractérisé
a) en ce que l'on introduit préalablement le composé de formule II et la solution de (p-toluènesulfonyl)-méthylisocyanure et
b) en ce que l'on ajoute l'hydroxyde de sodium en solution aqueuse.
